Europäisches Patentamt

European Patent Office

Office européen des brevets

(19)

(11) Veröffentlichungsnummer: **0 226 121 B1**

# EUROPÄISCHE PATENTSCHRIFT

(12)

(45) Veröffentlichungstag der Patentschrift: **22.01.92**

(51) Int. Cl.⁵: **C07H 15/22**

(21) Anmeldenummer: **86116773.2**

(22) Anmeldetag: **02.12.86**

(54) **Zubereitung enthaltend hochreine Acarbose.**

(30) Priorität: **13.12.85 DE 3543999**

(43) Veröffentlichungstag der Anmeldung:
**24.06.87 Patentblatt 87/26**

(45) Bekanntmachung des Hinweises auf die
Patenterteilung:
**22.01.92 Patentblatt 92/04**

(84) Benannte Vertragsstaaten:
**AT BE CH DE ES FR GB IT LI NL SE**

(56) Entgegenhaltungen:
**DE-A- 2 719 912**

**JOURNAL OF ANTIBIOTICS, Band 36, Nr. 9,
September 1983, Seiten 1166-1175, Tokyo,
JP; K. YOKOSE et al.: "New alpha-amylase
inhibitor, trestatins, II. Structure determination of trestatins A,B and C"**

**ALDRICH CATALOGUE/HANDBOOK OF FINE
CHEMICALS, Aldrich Chem. CO. LTD, Gillingham, GB, 1983-1984, Ref.Nr. 21635-6**

(73) Patentinhaber: **BAYER AG**

**W-5090 Leverkusen 1 Bayerwerk(DE)**

(72) Erfinder: **Rauenbusch, Erich, Dr.
Tersteegenweg 9
W-5600 Wuppertal 1(DE)**

# EP 0 226 121 B1

## Beschreibung

Die Erfindung betrifft Zubereitungen enthaltend hochreine Acarbose, ein Verfahren zu ihrer Herstellung sowie ihre Verwendung in und zur Herstellung von Arzneimitteln.

Acarbose ist ein Inhibitor des Saccharase Enzymkomplexes des menschlichen Dünndarms und wird in der Medizin zur Behandlung von Diabetes verwendet.

Acarbose ist O-4,6-Didesoxy-4-[[1S,4R,5S,6S]-4,5,6-trihydroxy-3-(hydroxymethyl)-2-cyclohexen-1-yl]-amino]-$\alpha$-D-glucopyranosyl-(1→4)-O-$\alpha$-D-glucopyranosyl-(1→4)-D-glucopyranose.

Der Inhibitor wird durch Fermentation von Actinoplanes Spezies gewonnen (s. DE-PS 2 209 832, DE-PS 2 209 834, DE-PS 2 064 092) und muß aus der Fermentationsbrühe isoliert werden. Hierzu wurden Reinigungsverfahren beschrieben (s. DE-PS 2 347 782 u. DE-PS 2 719 912).

Bei diesen Reinigungsverfahren wird die Acarbose an stark sauren Kationenaustauschern gebunden und mit Salzlösungen oder vorwiegend mit verdünnter Säure eluiert. Nach der Neutralisation mit Anionenaustauschern erhält man eine Acarbose mit einem Acarbose-Gehalt von 78 - 88 % in der Trockensubstanz (iT, HPLC-Methode). Als Verunreinigungen sind in diesen Präparationen noch etwa 10 - 15 % auf Zucker anfärbbare Nebenkomponenten, 1 - 4 % Asche und einige färbende Bestandteile enthalten. Für die humanmedizinische Anwendung werden noch höhere Reinheitsgrade benötigt, die sich jedoch bei Kenntnis des obengenannten Standes der Technik nicht einfach durch Ersatz der stark sauren Kationenaustauscher durch schwach saure Kationenaustauscher erreichen lassen, da diese Austauscher die sehr schwach basische Acarbose nicht genügend binden und sie ungereinigt im Effluat erscheint.

Das Journal of Antibiotics, Band 36, Nr. 9, September 1983, S. 1166 - 1175 beschreibt Strukturuntersuchungen and diversen $\alpha$-Amylase-Inhibitoren mit Hilfe von NMR. Die dort verwendete Acarbose wurde durch Hydrolyse von Trestatin gewonnen.

Überraschenderweise wurde nun gefunden, daß man die nach dem Stand der Technik vorgereinigte Acarbose doch an ganz bestimmten schwach sauren hydrophilen Kationenaustauschern in eng begrenzten pH-Bereichen in einem Schritt von restlichen Salzen, Farbstoffen und den zuckerhaltigen, basischen Nebenkomponenten reinigen kann. Der Gehalt an Acarbose steigt danach auf mindestens 90 Gew.-%, bevorzugt auf 95 - 98 Gew.-% und mehr an, die Sulfatasche sinkt auf 0 - 0,5 % ab und die zuckerartigen Nebenkomponenten gehen auf weniger als 10 Gew.-%, bevorzugt 2 - 5 Gew.-% und weniger zurück.

Die Erfindung betrifft daher Acarbosezubereitungen, enthaltend weniger als 10 Gew.-% zuckerartiger Nebenkomponenten

Bevorzugt ist Acarbose, enthaltend 2 bis 5 Gew.-% zuckerartiger Nebenkomponenten und ganz besonders bevorzugt betrifft die Erfindung Acarbose, enthaltend weniger als 2 Gew.-% zuckerartiger Nebenkomponenten.

Zur Herstellung der erfindungsgemäßen Acarbose mit dieser speziellen Art der Chromatographie wird eine Lösung der vorgereinigten Acarbose verwendet, die man zum Beispiel nach dem Verfahren, welches in der DE-PS 2 719 912 beschrieben wurde, erhält. Diese Lösung wird in einer Konzentration von 1 - 20 % und bei einem pH-Wert von 3,5 - 6,5, bevorzugt 4,0 - 5,5 auf eine Säule aufgetragen. Als Füllung eignen sich schwach saure Kationenaustauscher mit Carboxylgruppen auf der Basis von Dextran, Agarose und Cellulose, bzw. aus diesen Komponenten unter Zusatz von Polyacrylamiden abgeleitete Austauscher wie zum Beispiel die handelsüblichen Typen CM-Sephadex®, CM-Sepharose®, CM-Cellulose®, CM-Cellufine® u.a.. Merkwürdigerweise waren die handelsüblichen schwachsauren carboxylgruppenhaltigen Austauscher auf der Basis von Polystyrol, Polyacrylsäure oder Polymethacrylsäure für diese Reinigung nicht brauchbar.

Ein weiterer Gegenstand der Erfindung ist demgemäß auch ein Verfahren zur Herstellung von Acarbose, die weniger als 10 Gew.-% zuckerartiger Nebenkomponenten enthält, das dadurch gekennzeichnet ist, daß man vorgereinigte Acarbose in wäßriger 1 bis 20 gew.-%iger Lösung mit einem pH-Wert von 4 bis 7 auf eine Säule aufträgt, die als Füllmaterial schwach saure Kationenaustauscher mit Carboxylgruppen auf der Basis von Dextran, Agarose und Cellulose oder aus diesen unter Zusatz von Polyamid abgeleitete Austauscher enthält, die Säule ausschließlich mit entgastem, destilliertem Wasser eluiert und gegebenenfalls die Acarbose aus dem Eluat in üblicher Weise isoliert.

Die wäßrige Lösung der vorgereinigten Acarbose, die auf die Säule aufgetragen wird, ist in ihrem Volumen beschränkt. Man kann höchstens ein Volumen auftragen, welches dem Füllvolumen der Säule entspricht, vorzugsweise bleibt man unter 60 % des Säulenvolumens. Um eine präparative Menge von Acarbose zu reinigen, verwendet man deshalb keine zu niedrigen Konzentrationen. Nach oben ist die Konzentration dadurch beschränkt, daß die für die Reinigung am besten geeigneten Ionenaustauscher zur Schrumpfung neigen. Bevorzugt werden Konzentrationen von 7 - 20 %.

Nach dem Auftragen wird die Säule ausschließlich mit entgastem, destilliertem Wasser eluiert. Hierbei werden Salze, neutrale Zucker und färbende Begleitstoffe zuerst eluiert und nachfolgend verzögert die

2

Acarbose in einem verhältnismäßig breiten Maximum. Die zuckerartigen basischen Nebenkomponenten verbleiben auf der Säule und werden erst bei ihrer Regeneration entfernt. Die Acarbose liegt damit in einer rein wäßrigen Lösung bei einem pH-Wert von 6 - 7 vor und kann auf übliche Weise konzentriert und in hochreiner Form getrocknet werden.

Das Verhalten der Acarbose auf der Säule ist von mehreren Faktoren abhängig, von denen für die praktische Durchführung überraschenderweise der pH-Wert der Äquilibrierung der Säulenfüllung und die Temperatur während der Chromatographie entscheidend sind.

Mit dem pH-Wert der Säulenfüllung wird die Kapazität und das Elutionsverhalten der Acarbose verändert. Bei neutralen pH-Werten wird die Acarbose gegenüber den Salzen zu wenig verzögert und nicht genügend getrennt. Bei sauren pH-Werten um 3,5 - 6 wird die Acarbose stark verzögert und mit Wasser nur unvollständig eluiert. Die praktische Durchführung des Verfahrens benötigt für den jeweiligen Austauscher eine Optimierung des pH-Wertes. Im allgemeinen sind pH-Werte zwischen 4,3 und 5,0 geeignet. Bei hoher Belastung sind pH-Werte um 4,6, bei geringer Beladung und maximaler Ausbeute solche um 4,9 vorzuziehen.

Der zweite wichtige Faktor ist die Temperatur. Je niedriger die Temperatur ist, um so stärker wird die Acarbose von dem Ionenaustauscher zurückgehalten, um so größer ist die Kapazität der Säule, aber um so langsamer wird auch die Acarbose eluiert. Das heißt, man erhält ein asymmetrisches Maximum und ein sehr großes Volumen der Acarbosefraktion. Es ist damit günstig, die Substanz bei Raumtemperatur oder noch darunter aufzutragen und nach der Elution der Salze und färbenden Bestandteile die Säule auf etwa 25 bis 90° C, bevorzugt auf 40 - 70° C aufzuheizen. Man erreicht dadurch eine rasche Elution der Acarbose mit guten Ausbeuten.

Zur Regeneration der Ionenaustauscher werden Puffer, z.B. Natriumacetat-Puffer in dem für die Äquilibrierung notwendigen pH-Bereich und in einer Konzentration von 0,1 bis 0,5 M verwendet. Danach wird die Säule mit reinem, entgastem Wasser gewaschen bis die Leitfähigkeit auf etwa 0,1 mS/cm (Raumtemp.) abgefallen ist.

In den Abbildungen der Trennungen ist gegen die Zeit in Stunden auf der Abszisse der Brechungsindex des Eluats bzw. dessen Leitfähigkeit (gestrichelt) aufgetragen. Außerdem ist die Temperaturkennung angegeben.

Der Gehalt an Acarbose im Endprodukt wurde vor allen durch Flüssigkeitschromatographie (HPLC-Methode) bestimmt und auf die wasserfreie Substanz bezogen. Die Methode wurde wie folgt durchgeführt:

Hochdruckflüssigkeitschromatograph mit thermostatisiertem Säulenofen,
Metallsäule aus Edelstahl Länge: 25 cm
Innendurchmesser: 4 mm Füllung mit : Aminophase 5 $\mu$m
(z.B. LiChrosorb®NH$_2$, E.Merck, oder Hypersil®APS, Shandon)

| | |
|---|---|
| Reagenzien | 1. Acetonitril (z.B. LiChrosolv, E.Merck) |
| | 2. Kaliumdihydrogenphosphat zur Analyse |
| | 3. di-Natriumhydrogenphosphat-Dihydrat zur Analyse |
| Prüflösung | Etwa 200 mg Substanz, genau gewogen in einem Meßkolben mit Wasser zu 10,0 ml lösen. 20 mg/ml |
| Vergleichslösung | Inhalt einer Ampulle Standard-substanz in dem für den Standard angegebenen Volumen Wasser lösen. |
| Elutionsmittel | Acetonitril-Phosphatpuffer (71 + 29, Volumen), Phosphatpuffer: 600 mg Kaliumdihydrogenphosphat, und 350 mg di-Natriumhydrogenphosphat-Dihydrat mit Wasser zu 1000 ml lösen. Lösung über ein Millipore-Filter, Type AAWP 0,8 $\mu$m, filtrieren. |
| Durchflußrate | 2,2 ml/min. |
| Temperatur des Säulenofens | 35° C |
| Detektion | UV, 210 nm |
| Einspritzmenge | 10 $\mu$l, 0,2 mg in 10 $\mu$l |
| Schreiber-Vollausschlag | ca. 0,25 AUFS (absorbance units full scale) |
| Berechnung des Acarbose-Gehaltes | |

$$G = \frac{P_p \times C \times 100\ 000}{P_v \times W_p \times (100-b)}$$

| | | |
|---|---|---|
| G = | | Gehalt Acarbose in Prozent, berechnet auf die wasserfreie Substanz |
| $P_p$ = | | Peakfläche der Acarbose aus der Prüflösung |
| $P_v$ = | | Peakfläsche der Acarbose aus der Vergleichslösung (Standard) |
| $W_p$ = | | Einwaage der Probe in mg |
| C = | | Konzentration der Vergleichslösung in mg Acarbose pro ml |
| b = | | Wassergehalt der Probe in Prozent |

Die Inhibitorwirkung der Acarbose wurde im Saccharase-Inhibitionstest bestimmt und Saccharase-Inhibitions-Einheiten (SIE) angegeben. Der Test ist von L. Müller, B. Junge et al. in Enzyme Inhibitors, U. Brodbeck ed., Verlag Chemie, 1980, S. 109, beschrieben.

Zur vorliegenden Erfindung gehören pharmazeutische Zubereitungen, die neben nichttoxischen, inerten pharmazeutisch geeigneten Trägerstoffen den erfindungsgemäßen Wirkstoff enthalten oder die aus dem erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten, Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierrungsmittel enthaltenden Überzügen und Hüllen versehen sein und auch so zusammengesetzt sein, daß sie den Wirkstoff nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem Wirkstoff die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem Wirkstoff die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem Wirkstoff die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem Wirkstoff die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem Wirkstoff die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessende Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung des erfindungsgemäßen Wirkstoffs zur Herstellung von pharmazeutischen Zubereitungen. Diese dienen in der Human- und Veterinärmedizin zur Verhütung, Besserung und/oder Heilung von Erkrankungen.

Der Wirkstoff oder seine pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den erfindungsgemäßen Wirkstoff in Gesamtmengen von etwa 1 bis etwa 40, vorzugsweise 2 bis 8 mg/kg Körpergewicht je 24 Stunden, gegebenenfalls in Form mehrerer Einzelgaben zur Erzielung der gewünschten Ergebnisse zu verabreichen.

Eine Einzelgabe enthält den erfindungsgemäßen Wirkstoff, vorzugsweise in Mengen von etwa 0,1 bis etwa 4, insbesondere 0,2 bis 2 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkranung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart des Wirkstoffs kann durch jeden Fachmann aufgrund seines Fachwissens leicht erfolgen.

Beispiel 1

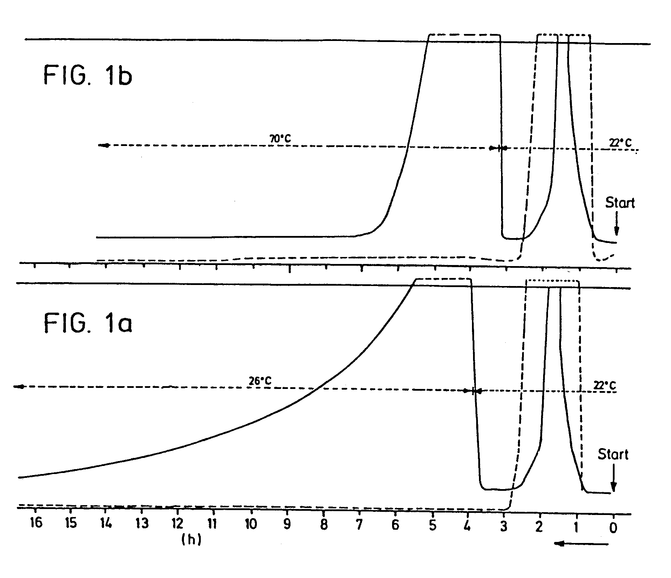

Eine Chromatographie-Säule mit 2,6 cm Durchmesser und 40 cm Länge (Pharmacia K 26/40) wurde mit CM-Sephadex® C 25 gefüllt. Das CM-Sephadex® C 25 war zuvor in 0,2 M-Natrium-acetat-Puffer pH 4,7 äquilibriert worden. Nach der Füllung wurde die Säule mit destilliertem, entgastem Wasser gewaschen bis die Leitfähigkeit auf 0,1 mS/cm abgefallen war. Die Betthöhe in der Säule betrug dann 34 cm. Als Testsubstanz wurden 9,2 g einer vorgereinigten Acarbose verwendet, welche neben Wasser noch Salze und andere Verunreinigungen enthielt. Die Acarbose wurde in etwa 40 ml dest. Wasser gelöst, der pH-Wert durch Zugabe von etwas Salzsäure auf 4,7 eingestellt und die Lösung auf 50 ml aufgefüllt. Der Inhibitorgehalt betrug 446 550 SIE entsprechend 5,75 g Reinacarbose wasserfrei. Die Substanz wurde mit einer Durchflußgeschwindigkeit von 100 ml/h (18,8 cm/h) auf die Säule aufgetragen und mit destilliertem Wasser von 26°C nachgewaschen und eluiert. Der Verlauf der Trennung ist in Fig. 1a wiedergegeben. Die Hauptfraktion wurde vereinigt und ergab eine Ausbeute von 5,87 g mit 399 300 SIE, das sind 89 % des eingesetzten Inhibitors. Die spezifische Aktivität betrug 72 SIE/mg in der Trockensubstanz. Die HPLC-Methode ergab einen Gehalt von 93 % i.T.

Die Regeneration der Säule wurde mit 800 ml 0,2 M-Natriumacetat-Puffer pH 4,7 vorgenommen und diese anschließend mit 600 ml destilliertem, entgastem Wasser ausgewaschen.

Beispiel 2 - 5

Entsprechend dem Beispiel 1 wurden alle Beispiele der Tabelle 1 durchgeführt, wobei jedoch die Temperatur des Säulenmantels während der Elution variiert wurde. Es wurde so vorgegangen, daß 3 Stunden nach Beginn der Auftragung der Thermostat der Säulenheizung eingeschaltet wurde, der je nach eingestellter Temperatur den Sollwert in 3 - 12 Minuten erreicht hat. Der Rückgang des Volumens der Hauptfraktion ist in der Tabelle 1 angegeben und das Beispiel 5 mit einer Elutionstemperatur von 70°C in der Fig. 1b.

## Tabelle 1

Acarbose- Chromatographie an CM-Sephadex® C 25

Abhängigkeit des Elutionsvolumens von der Temperatur

| Beispiel | Temperatur der Elution | Volumen der Haupt- fraktion | Ausbeute | | Gehalt nach HPLC |
|---|---|---|---|---|---|
| | °C | ml | g | % | % |
| 1 | 26 | 1 163 | 5,87 | 89 | 93 |
| 2 | 40 | 840 | 6,25 | 100 | 92 |
| 3 | 50 | 570 | 6,04 | 91 | 90 |
| 4 | 60 | 460 | 5,92 | 86 | 92 |
| 5 | 70 | 380 | 6,62 | 99 | 94 |

Beispiel 6

Eine Chromatographiesäule (Pharmacia K 26/70 wurde wie in Beispiel 1 mit CM-Sephadex® C 25 gefüllt, die jedoch auf pH 4,3 äquilibriert und gewaschen worden war, Die Füllhöhe betrug 47 cm. Als Testsubstanz wurde wieder wie in Beispiel 1 eine vorgereinigte Acarbose verwendet, wobei 579 000 SIE in 200 ml Wasser aufgetragen wurden. Der Durchfluß betrug 117 ml/h (22 cm/h). Die Elution erfolgte mit Wasser; wie bei Beispiel 1 wurden zuerst salzhaltige Fraktionen eluiert und anschließend die Acarbose. Der Abstand zwischen dem Ende der salzhaltigen Fraktionen und dem Beginn des Anstiegs der Acarbose betrug 162 ml. Die Elution der Acarbose wurde durch Erhitzen der Säule auf 45°C beschleunigt. Das Volumen der Acarbosefraktion betrug 1048 ml, die Ausbeute betrug 577 000 SIE, das sind 100 % der aufgetragenen Menge.

Beispiel 7

Wie in Beispiel 6 wurde eine Chromatographiesäule mit CM-Sephadex® C 25 gefüllt, welches jedoch auf pH 4,9 äquilibriert und gewaschen worden war, 200 ml der Testlösung mit 579 000 SIE wurden aufgetragen und die Substanzen mit Wasser eluiert. Zwischen den salzhaltigen Fraktionen und dem Beginn der Acarbosefraktion war nur noch ein Abstand von 23 ml. Das Volumen der Hauptfraktion betrug 707 ml, wobei wieder auf 45°C erhitzt wurde, Die Ausbeute betrug 577 000 SIE, das sind 100 % der aufgetragenen Menge.

Beispiel 8

Eine Chromatographie-Säule (Pharmacia K 26/40) wurde mit CM-Sepharose® C1 6B fast flow gefüllt, auf pH 4,5 mit 0,2 M-Natrium-acetat-Lösung äquilibriert und mit Wasser ausgewaschen. 40,5 ml einer Lösung vorgereinigter Acarbose mit einer Hemmwirkung von 247 300 SIE wurden aufgetragen. Die Durchflußgeschwindigkeit betrug 100 ml/h (18,8 cm/h). Die Elution erfolgte mit entgastem, destilliertem Wasser, wobei die Säule mit Beginn der Acarbose-Elution auf 45°C aufgeheizt wurde. Der Abstand zwischen der Salz- und der Acarbosefraktion betrug 38 ml, das Volumen der Hauptfraktion 600 ml. Die Ausbeute an Acarbose betrug 247 000 SIE, das sind 100 % der eingesetzten Menge. Gehalt nach HPLC-Methode 98 % i.T.

Beispiel 9

Eine Chromatographiesäule nach Beispiel 8 wurde mit Carboxymethylcellulose CM 52® (Whatman) gefüllt, mit 0,2 M-Natrium-acetat-Lösung auf pH 4,5 äquilibriert und mit Wasser ausgewaschen. Die Füllhöhe betrug 36 cm. 62 ml einer Lösung von vorgereinigter Acarbose mit einer Hemmwirkung von 394 000 SIE wurden aufgetragen und wie in Beispiel 8 eluiert. Die Acarbosefraktion folgte unmittelbar der Salzfraktion. Das Volumen der Acarbosefraktion betrug 850 ml, die Ausbeute 322 000 SIE, das sind 82 % der eingesetzten Menge. Gehalt nach HPLC-Methode 90 % i.T.

Beispiel 10

Eine Chromatographiesäule nach Beispiel 8 wurde mit Matrex-Cellufine CM® (Amicon) gefüllt, mit 0,2 M-Natriumacetat-Lösung auf pH 4,5 äquilibriert und mit Wasser ausgewaschen. Die Füllhöhe betrug 37 cm. 62 ml einer Lösung von vorgereinigter Acarbose mit einer Hemmwirkung von 394 000 SIE wurden aufgetragen und wie in Beispiel 8 eluiert. Die Acarbosefraktion folgte der Salzfraktion in einem Abstand von 23 ml. Das Volumen der Acarbosefraktion betrug 960 ml, die Ausbeute 350 000 SIE, das sind 89 % der aufgetragenen Menge. Gehalt nach der HPLC-Methode 98 % i.T.

**Patentansprüche**
**Patentansprüche für folgende Vertragsstaaten: BE, CH, DE, FR, GB, IT, Li, NL, SE**

1.  Zubereitung, die Acarbose mit einem Gehalt von mindestens 90 Gew.-%, zuckerartige Nebenkomponente von weniger als 10 Gew.-% außer 0 % und gegebenenfalls Wasser enthält erhältlich dadurch, daß man vorgereinigte Acarbose in wäßriger 1 bis 20 gew.-%iger Lösung mit einem pH-Wert von 4 bis 7 auf eine Säule aufträgt, die als Füllmaterial schwach saure Kationenaustauscher mit Carboxylgruppen auf der Basis von Dextran, Agarose und Cellulose oder aus diesen unter Zusatz von **Polyacrylamid** abgeleitete Austauscher enthalten, die Säule ausschließlich mit entgastem, destilliertem Wasser eluiert und gegebenenfalls die Acarbose aus dem Eluat in üblicher Weise isoliert.

2.  Zubereitung nach Anspruch 1, die Acarbose mit einem Gehalt von 95 Gew.-% enthält.

3.  Zubereitung nach Anspruch 1, die Acarbose mit einem Gehalt von 98 Gew.-% enthält.

4.  Zubereitung gemäß den Ansprüchen 1 bis 3, dadurch gekennzeichnet, daß man ein Volumen der vorgereinigten Acarboselösung aufträgt, das dem Füllvolumen der Säule entspricht.

5.  Zubereitung gemäß den Ansprüchen 1 bis 3, dadurch gekennzeichnet, daß das Volumen der vorgereinigten Acarboselösung unterhalb 60 % des Säulenvolumens liegt.

6.  Zubereitung gemäß den Ansprüchen 1 bis 5, dadurch gekennzeichnet, daß der pH-Wert der vorgereinigten Acarboselösung 4,3 bis 5 beträgt.

7.  Zubereitung gemäß den Ansprüchen 1 bis 6, dadurch gekennzeichnet, daß man die vorgereinigte Acarboselösung bei Temperaturen bis zur Raumtemperatur, vorzugsweise im Bereich von 4 bis 25°C, aufträgt und nach der Elution der Salze und färbenden Bestandteile die Säule auf 25 bis 90°C, vorzugsweise auf 40 bis 70°C, aufheizt.

8.  Arzneimittel, enthaltend eine Zubereitung gemäß den Ansprüchen 1 bis 7.

9.  Verwendung von einer Zubereitung gemäß den Ansprüchen 1 bis 7 zur Herstellung von Arzneimitteln.

**Patentansprüche für folgende Vertragsstaaten: AT, ES**

1. Verfahren zur Herstellung einer Zubereitung, die Acarbose mit einem Gehalt von mindestens 90 Gew.-%, zuckerartige Nebenkomponente von weniger als 10 Gew.-% außer 0 % und gegebenenfalls Wasser enthält, dadurch gekennzeichnet, daß man vorgereinigte Acarbose in wäßriger 1 bis 20 gew.-%iger Lösung mit einem pH-Wert von 4 bis 7 auf eine Säule aufträgt, die als Füllmaterial schwach saure Kationenaustauscher mit Carboxylgruppen auf der Basis von Dextran, Agarose und Cellulose oder aus diesen unter Zusatz von Polyacrylamid abgeleitete Austauscher enthalten, die Säule ausschließlich mit entgastem, destilliertem Wasser eluiert und gegebenenfalls die Acarbose aus dem Eluat in üblicher Weise isoliert.

2. Verfahren nach Anspruch 1, dadurch gekennzeichnet, daß man ein Volumen der vorgereinigten Acarboselösung aufträgt, das dem Füllvolumen der Säule entspricht.

3. Verfahren nach Anspruch 2, dadurch gekennzeichnet, daß das Volumen der vorgereinigten Acarboselösung unterhalb 60 % des Säulenvolumens liegt.

4. Verfahren nach den Ansprüchen 1 bis 3, dadurch gekennzeichnet, daß der pH-Wert der vorgereinigten Acarboselösung 3,5 bis 6,0, vorzugsweise 4,3 bis 5,0 beträgt.

5. Verfahren nach den Ansprüchen 1 bis 4, dadurch gekennzeichnet, daß man die vorgereinigte Acarboselösung bei Temperaturen bis zur Raumtemperatur, vorzugsweise im Bereich von 4 bis 25°C, aufträgt und nach der Elution der Salze und färbenden Bestandteile die Säule auf 25 bis 90°C, vorzugsweise auf 40 bis 70°C, aufheizt.

**Claims**

**Claims for the following Contracting States: BE, CH, DE, FR, GB, IT, LI, NL, SE**

1. Preparation which contains acarbose with a content of not less than 90% by weight, sugar-like secondary components of less than 10% by weight except 0% and, where appropriate, water, obtainable by applying prepurified acarbose in a 1 to 20% by weight aqueous solution with a pH of 4 to 7 to a column which contains as packing material weakly acid cation exchangers which have carboxyl groups and are based on dextran, agarose and cellulose or exchangers which are derived from the latter with the addition of polyacrylamide, eluting the column exclusively with degassed, distilled water and, where appropriate, isolating the acarbose from the eluate in customary manner.

2. Preparation according to Claim 1, which contains acarbose with a content of 95% by weight.

3. Preparation according to Claim 1, which contains acarbose with a content of 98% by weight.

4. Preparation according to Claims 1 to 3, characterised in that the volume of prepurified acarbose solution which is applied corresponds to the filling volume of the column.

5. Preparation according to Claims 1 to 3, characterised in that the volume of the prepurified acarbose solution is less than 60% of the column volume.

6. Preparation according to Claims 1 to 5, characterised in that the pH of the prepurified acarbose solution is 4.3 to 5.

7. Preparation according to Claims 1 to 6, characterised in that the prepurified acarbose solution is applied at temperatures up to room temperature, preferably in the range from 4 to 25°C, and, after the salts and colouring constituents have been eluted, the column is heated to 25 to 90°C, preferably to 40 to 70°C.

8. Medicaments containing a preparation according to Claims 1 to 7.

9. Use of a preparation according to Claims 1 to 7 for the preparation of medicaments.

**Claims for the following Contracting States: AT, ES**

1. Process for preparing a preparation which contains acarbose with a content of not less than 90% by weight, sugar-like secondary components of less than 10% by weight except 0% and, where appropriate, water, characterized in that prepurified acarbose in a 1 to 20% by weight aqueous solution with a pH of 4 to 7 is applied to a column which contains as packing material weakly acid cation exchangers which have carboxyl groups and are based on dextran, agarose and cellulose or exchangers which are derived from the latter with the addition of polyacrylamide, the column is eluted exclusively with degassed, distilled water and, where appropriate, the acarbose is isolated from the eluate in customary manner.

2. Process according to Claim 1, characterised in that the volume of prepurified acarbose solution which is applied corresponds to the filling volume of the column.

3. Process according to Claim 2, characterized in that the volume of the prepurified acarbose solution is less than 60% of the column volume.

4. Process according to Claims 1 to 3, characterised in that the pH of the prepurified acarbose solution is 3.5 to 6.0, preferably 4.3 to 5.0.

5. Process according to Claims 1 to 4, characterised in that the prepurified acarbose solution is applied at temperatures up to room temperature, preferably in the range from 4 to 25°C, and, after the salts and colouring constituents have been eluted, the column is heated to 25 to 90°C, preferably 40 to 70°C.

**Revendications**
**Revendications pour les Etats contractants suivants: BE, CH, DE, FR, GB, IT, LI, NL, SE**

1. Composition contenant de l'acarbose à une teneur d'au moins 90 % en poids, des produits d'accompagnement du type sucres à une teneur inférieure à 10 % en poids mais non à 0 %, et le cas échéant de l'eau, obtenue en jetant de l'acarbose purifié au préalable, en solution aqueuse à une concentration de 1 à 20 % en poids et à un pH de 4 à 7, sur une colonne contenant en tant que matières de garnissage des échangeurs de cations faiblement acides à groupes carboxyle à base de dextranne, d'agarose et de cellulose ou de tels échangeurs additionnés d'échangeurs dérivés d'un polyacrylamide, en éluant la colonne exclusivement par de l'eau distillée dégazée et le cas échéant en isolant l'acarbose de l'éluat de la manière habituelle.

2. Composition selon la revendication 1, contenant l'acarbose à une teneur de 95 % en poids.

3. Composition selon la revendication 1, contenant l'acarbose à une teneur de 98 % en poids.

4. Composition selon les revendications 1 à 3, caractérisée en ce que l'on applique sur la colonne un volume de la solution d'acarbose purifié au préalable correspondant au volume de garnisage de la colonne.

5. Composition selon les revendications 1 à 3, caractérisée en ce que le volume de la solution d'acarbose purifié au préalable représente moins de 60 % du volume de la colonne.

6. Composition selon les revendications 1 à 5, caractérisée en ce que le pH de la solution d'acarbose purifié au préalable est de 4,3 à 5.

7. Composition selon les revendications 1 à 6, caractérisée en ce que la solution d'acarbose purifié au préalable est appliquée sur colonne à des températures allant jusqu'à la température ambiante, de préférence à des températures dans l'intervalle de 4 à 25°C, et la colonne est chauffée à une température de 25 à 90°C, de préférence de 40 à 70°C, après élution des sels et des constituants colorants.

8. Médicament contenant une composition selon les revendications 1 à 7.

9. Utilisation d'une composition selon les revendications 1 à 7 pour la préparation de médicaments.

**EP 0 226 121 B1**

**Revendications pour les Etats contractants suivants: AT, ES**

1. Procédé de préparation d'une composition contenant l'acarbose à une teneur d'au moins 90 % en poids, des produits d'accompagnement du type sucres à une teneur inférieure à 10 % en poids mais non à 0 %, et le cas échéant de l'eau, caractérisé en ce que l'on jette l'acarbose, purifié au préalable, en solution aqueuse à une concentration de 1 à 20 % en poids, à un pH de 4 à 7, sur une colonne contenant en tant que matières de garnissage des échangeurs de cations faiblement acides à groupes carboxyle, à base de dextranne, d'agarose et de cellulose ou de tels échangeurs additionnés d'échangeurs dérivés d'un polyacrylamide, on élue la colonne exclusivement avec de l'eau distillée dégazée et le cas échéant on isole l'acarbose de l'éluat de la manière habituelle.

2. Procédé selon la revendication 1, caractérisé en ce que l'on jette sur colonne un volume de la solution d'acarbose purifié au préalable correspondant au volume de garnissage de la colonne.

3. Procédé selon la revendication 2, caractérisé en ce que le volume de la solution d'acarbose purifié au préalable représente moins de 60 % du volume de la colonne.

4. Procédé selon les revendications 1 à 3, caractérisé en ce que le pH de la solution d'acarbose purifié au préalable est de 3,5 à 6,0, de préférence de 4,3 à 5,0.

5. Procédé selon les revendications 1 à 4, caractérisé en ce que la solution d'acarbose purifié au préalable est jetée sur colonne à des températures allant jusqu'à la température ambiante, de préférence dans l'intervalle de 4 à 25 ° C, et la colonne est chauffées à des températures de 25 à 90 ° C, de préférence de 40 à 70 ° C après élution des sels et des constituants colorants.

FIG. 1b

70°C

22°C

Start

FIG. 1a

26°C

22°C

Start

16  15  14  13  12  11  10  9  8  7  6  5  4  3  2  1  0

(h)